(19) Europäisches Patentamt
European Patent Office
Office européen des brevets

(11) Numéro de publication: **0 236 187**
**A1**

(12) **DEMANDE DE BREVET EUROPEEN**

(21) Numéro de dépôt: **87400225.6**

(22) Date de dépôt: **02.02.87**

(51) Int. Cl.⁴: **C 07 K 5/06**, C 07 D 405/14, A 61 K 31/495

(30) Priorité: **04.02.86 FR 8601496**

(43) Date de publication de la demande: **09.09.87 Bulletin 87/37**

(84) Etats contractants désignés: **AT BE CH DE ES FR GB GR IT LI LU NL SE**

(71) Demandeur: **IRE-CELLTARG S.A., Zone Industrielle, B-6220 Fleurus (BE)**

(72) Inventeur: **Le Clef, Brigitte, Rampe de Floribois 2, B-1348 Louvain-La-Neuve (BE)**
Inventeur: **Laub, Ruth, Avenue Besme 6, B-1190 Bruxelles (BE)**
Inventeur: **Schneider, Yves-Jacques, Dennenboslaan 14, B-1900 Overijse (BE)**

(74) Mandataire: **Warcoin, Jacques et al, Cabinet Régimbeau 26, avenue Kléber, F-75116 Paris (FR)**

(54) **Dérivés d'imidazoles, procédé de préparation et compositions pharmaceutiques les contenant.**

(57) La présente invention concerne des composés de formule

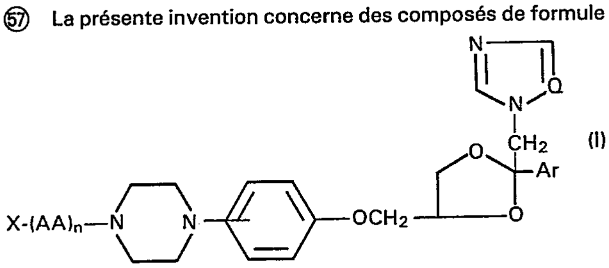

(I)

dans laquelle
Q est N ou CH,
Ar est un radical choisi parmi phényle et thienyle éventuellement substitué, les substituants pouvant être de un à trois substituants halo, alkyle inférieur ou alkoxy inférieur,
-AA- représente un radical divalent correspondant à un amino acide entrant dans la constitution des protéines, certaines fonction de ce radical pouvant être protégées, ou bien représente un radical diacyle,
n est un entier de 0 à 4 inclus, et

X représente H, OH ou Y, 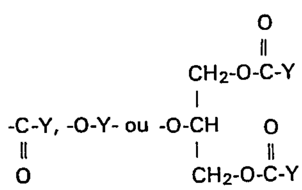 où

lorsque n est égal à 1, 2, 3 ou 4, alors Y est un radical hydrocarboné ayant de 1 à 20 atomes de carbone,
lorsque n est égal à 0, alors Y est un radical hydrocarboné ayant de 7 à 20 atomes de carbone,
ainsi que les sels et les isomères de ces composés,
un procédé de préparation de ces composés, leur application comme agents fongicides et/ou bactéricides et leurs compositions les comportant.

EP 0 236 187 A1

DERIVES D'IMIDAZOLES, PROCEDE DE PREPARATION ET COMPOSITIONS PHARMACEUTIQUES LES CONTENANT.

La présente invention concerne des dérivés de (1,3-dioxolane-2-yl-méthyl)-1H- imidazole ayant des propriétés fongicides et bactéricides, un procédé de préparation, des intermédiaires de synthèse de ces composés, et des compositions bactéricides et/ou fongicides comportant ces dérivés.

Plus particulièrement, il s'agit de composés de formule :

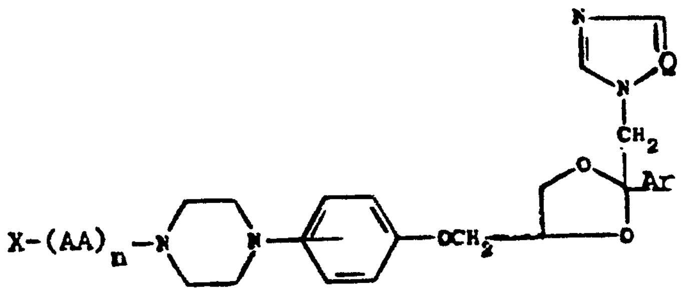

(I)

dans laquelle :

Q est N ou CH ;

Ar est un radical choisi parmi phényle et thiényle éventuellement substitué, les substituants pouvant être de un à trois substituants halo, alkyle inférieur ou alkoxy inférieur;

-AA- représente un radical divalent correspondant à un aminoacide entrant dans la constitution des protéines, certaines fonction de ce radical pouvant être protégées, ou bien représente un radical diacyle;

n est un entier de 0 à 4 inclus; et

X représente H, OH ou Y, $-\underset{\underset{O}{\|}}{C}-Y$, -O-Y ou $-O-\begin{array}{l} CH_2O-\overset{\overset{O}{\|}}{C}-Y \\ CH \\ CH_2O-\underset{\underset{O}{\|}}{C}-Y \end{array}$ ,où

lorsque n est égal à 1,2,3 ou 4 alors Y est un radical hydrocarboné ayant de 1 à 20 atomes de carbone,

lorsque n est égal à 0, alors Y est un radical hydrocarboné ayant de 7 à 20 atomes de carbone,

ainsi que les sels et les isomères de ces composés.

Les composés selon l'invention diffèrent des dérivés connus du kétoconazole par leur substituant en position 1 de la pipérazine.

Il s'agit notamment des composés de formule Ia

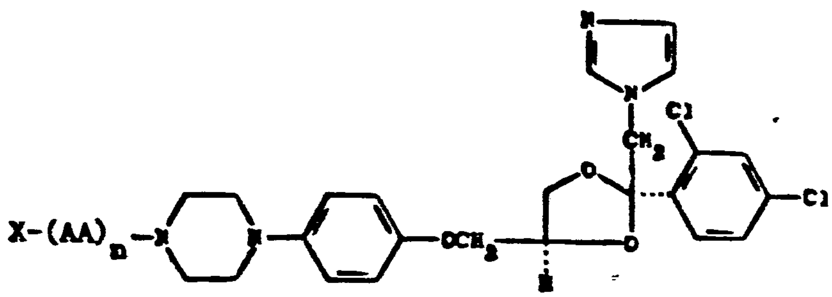 (Ia)

dans laquelle

- AA - représente $-\overset{O}{\overset{\|}{C}}-\underset{R}{\underset{|}{CH}}-NH-$,

où R représente les substituants latéraux en α du groupe amino des acides aminés ou le radical diacyle succ ;

n représente un entier de O à 4 inclus ;

X représente H, OH, Y, $-\underset{O}{\underset{\|}{C}}-Y$, -OY ou

$$\begin{array}{l} \quad CH_2O-\overset{O}{\overset{\|}{C}}-Y \\ \quad | \\ -O-CH \\ \quad | \\ \quad CH_2O-\overset{O}{\overset{\|}{C}}-Y \end{array}$$ , où

Y représente un radical hydrocarboné en $C_7$ à $C_{20}$, alkyle ou alcényle,

et succ représente le radical $-\overset{O}{\overset{\|}{C}}-CH_2-CH_2-\overset{O}{\overset{\|}{C}}-$,

et lorsque n ≠ O, alors Y peut être alkyle inférieur, leurs sels pharmaceutiquement acceptables et leurs formes isomères.

Dans la présente description, les radicaux alkyl et alkoxy inférieurs comportent de 1 à 6 atomes de carbone,et de préférence de 1 à 3 atomes de carbone. Le terme halo représente les radicaux dérivés des halogènes fluoro, chloro, bromo et iodo ainsi que le radical trifluorométhyle,

Les radicaux divalent -AA- correspondant à un amino acide entrant dans la constitution des protéines peuvent provenir d'un amino-acide sous forme D ou sous forme L,s'il existe un atome de carbone asymétrique. Le radical -AA- est fixé sur le radical pipérazine de préférence par son extrémité -C- terminale et le radical X est fixé de préférence sur l'extrémité N-terminale. Lorsque le radical -AA- comporte d'autres fonctions -OH, $-NH_2$, -COOH par exemple, celles-ci peuvent être libres ou protégées par un radical Pr connu dans la chimie des protéines, par exemple Cbz.

Les biradicaux -AA- tels qu'ils sont définis représentent tous les biradicaux dérivés des acides aminés, c'est-à-dire les acides aminés neutres et hydrophobes, comme les : glycine, alanine, valine, leucine, isoleucine, phénylalanine, proline et methionine, les acides aminés neutres et polaires comme les : serine, thréonine, tyrosine, tryptophane, asparagine, glutamine et cystéine, mais également les basiques comme les : lysine, arginine, histidine, et les acides comme l'acide aspartique et l'acide glutamique.

Le radical divalent -AA- peut être également le radical diacyle provenant d'un diacide alcandioïque ou alcendioïque comportant de 3 à 10 atomes de carbone,et éventuellement des substituants tels que par exemple les acides succiniques ou maléïques.

Lorsque n est égal à 2,3 ou 4,alors les radicaux-AA- peuvent être identiques ou différents. Selon l'invention, n est de préférence égal à 0, 1 ou 2.

Parmi les radicaux hydrocarbonés représentés par Y, il faut citer les radicaux alkyles, alcényles et alcynyles qui peuvent éventuellement être substitués. Ces radicaux peuvent être droits ou ramifiés et les radicaux alcényles peuvent comporter une ou plusieurs doubles liaisons.

Lorsque le radical Y comporte plus de 7 atomes de carbone, il s'agit de préférence du radical provenant d'acide gras naturel Y-COOH, par exemple les acides myristique, palmitique, stéarique, oléique, linoléique et arachidique.

Parmi les composés de formule Ia, il faut citer les composés dans lesquels :

n = O, X = $-\underset{\underset{O}{\|}}{C}-Y$, Y dérivant d'un acide gras,

n = 1,2, X = OH et le substituant en position 1 est -AA- succ-
-AA- dérivant de Ala ou Gly,

n = 1 X = $-\underset{\underset{O}{\|}}{C}-Y$, Y dérivant d'acide gras
et R = H ou $CH_3$.

Les carbones 2 et 4 du noyau dioxolane sont asymétriques, et sont donc des centres de stéréoisomérie.

La présente invention concerne toutes les formes stéréoisomères des composés de formule I, mais en particulier les composés ayant la configuration cis.

La présente invention concerne également les sels pharmaceutiquement acceptables des composés de formule I, par exemple leurs sels d'addition avec des acides ou des bases.

La présente invention concerne également un procédé de préparation du composé de formule I, caractérisé en ce que

on fait réagir le composé de formule

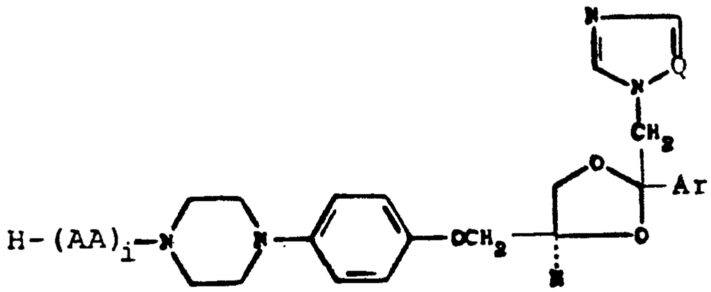

II

avec un acide de la formule

X-(AA)$_{n-i}$-H III1

où i est un entier et O ⩽ i ⩽ n, ou un dérivé réactif de ce composé, éventuellement en présence d'un auxiliaire de réaction, dans un solvant approprié et dans des conditions de température appropriées, les composés II et III étant éventuellement sous forme de dérivé réactif de la fonction acide ou amine et les fonctions non réactives pouvant être protégées. Et on libère éventuellement les groupements protégés, les dérivés réactifs des acides pouvant être des halogénures, chlorure par exemples, anhydride ester azido acyle. Les groupes de protection des acides ou des fonctions amines sont connus dans la synthèse des peptides.

Plus particulièrement, la présente invention concerne également un procédé de préparation du composé de formule I, caractérisé en ce que :

a) on fait réagir le composé de formule

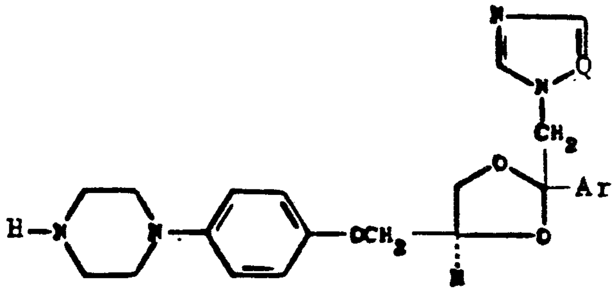

IV

avec un acide de la formule

$$X-(AA)_n-H \qquad V$$

ou un dérivé réactif de cet acide carboxylique, ou

b) on fait réagir le composé de formule

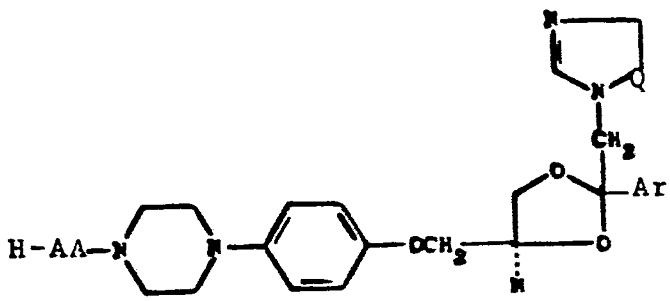

VI

avec un composé de formule

$$X-(AA)_{n-1}-H \qquad VII$$

ou un dérivé réactif de ce composé, ou éventuellement en présence d'un auxiliaire de réaction, dans un solvant approprié et dans des conditions de température appropriées, les composés VI et VII étant éventuellement sous forme de dérivé réactif de la fonction acide ou amine et les fonctions non réactives pouvant être protégées.

Ainsi, pour fixer un acide aminé soit directement en position 1 du cycle pipérazine, soit à la suite d'un ou des acides aminés déjà fixés, on fait réagir un dérivé approprié de l'acide aminé, dont la fonction amine est protégée, sur le dérivé de formule II, IV ou VI hydrogéné en bout de chaîne en position 1 de la pipérazine.

Ainsi, pour fixer la glycine ou l'alanine, on utilise un glycinate ou alaninate, par exemple le N-fluorényl-méthyloxycarbonylglycinate de N-hydroxy-succinimide.

A la fin de la réaction, on a fixé l'acide aminé en position 1, le groupe amino étant encore protégé par le groupe protecteur, par exemple le N-fluorenylméthyloxycarbonyle. On déprotège alors le composé obtenu par exemple à l'aide de pipéridine.

D'autres couples groupe protecteur-agent de déprotection sont bien sûr envisageables.

La réaction de fixation a lieu dans un solvant approprié, par exemple l'acétate d'éthyle, quoique d'autres solvants soient également envisageables.

Pour fixer un groupe du type diacyle, par exemple succinyle, toujours en position 1 de la pipérazine ou en bout de chaîne fixée à la pipérazine, on fait réagir un composé de formule II, IV ou VI tel que défini ci-dessus avec un dérivé carboxylique, en particulier avec un anhydride, succinique lorsque l'on veut fixer le radical de type succinique, ou lorsqu'on veut fixer par exemple un radical alcanoyle, l'anhydride de l'acide correspondant.

La réaction a lieu en température ambiante, dans des conditions pratiquement équimolaires, et en présence d'un solvant approprié, par exemple la diméthylformamide.

Selon un autre mode de réalisation, pour fixer par exemple un alcénoyle, on utilise un halogénure d'acide, par exemple le chlorure d'acide correspondant, dans un solvant approprié, la réaction a lieu alors en présence d'un auxiliaire de réaction, capable de fixer l'halogénure par exemple la triéthylamine, et dans des conditions de température appropriées, c'est-à-dire que le mélange est refroidi dans la glace au moment de l'addition de l'halogénure d'alcénoyle ajouté peu à peu. Là aussi, les conditions sont pratiquement équimolaires.

Quel que soit le mode de mise en oeuvre du procédé, pour isoler les produits, avant ou après une éventuelle déprotection du groupe aminé, on utilise les techniques classiques dévaporation du solvant, de lavage à l'eau ou à d'autres solvants, d'extraction à l'aide d'autres solvants, comme le chloroforme ou l'éther, de chromatographie ou de purification sur gel de silice et/ou de recristallisation.

L'invention concerne en outre les composés de formule II, IV ou VI tels que définis ci-dessus, et, à titre d'intermédiaire de synthèse pour la mise en oeuvre de procédé ci-dessus.

Les composés de formule II, IV ou VI sont connus en particulier par les brevets EP 0006722 et FR 2 378 778 où ils peuvent être préparés à partir de composés connus.

La Demanderesse a mis en évidence l'activité antibactérienne, fongicide et antitumorale des composés selon l'invention, comme cela apparaîtra dans les exemples.

C'est pourquoi, la présente invention concerne également à titre de médicaments nouveaux et à titre d'agents bactéricides et/ou fongicides et/ou antitumoraux, les composés de formule I et Ia définis ci-dessus.

C'est pourquoi la présente invention concerne également les compositions bactéricides et/ou fongicides et/ou antitumorales comportant au moins une quantité efficace d'un composé de formule I, ou un sel ou un de leur stéroisomère, et un support acceptable.

En particulier, la présente invention concerne les compositions pharmaceutiques comportant au moins une quantité efficace d'un composé selon l'invention, un sel pharmaceutiquement acceptable d'un de ses composés ou un de ses stéréoisomères et un support pharmaceutiquement acceptable.

Comme cela apparaîtra dans les exemples, les dérivés comportant des acides aminés montrent une activité comparable voire supérieure aux dérivés connus sur les champignons (en particulier le dérivé alanine) et sur le Staphilococus aureus (en particulier le dérivé glycyl).

Les dérivés du type succinyle sont particulièrement intéressants pour leur instabilité à pH acide, pH

comparable à celui des endosomes et lysosomes.

Parmi ces dérivés succinyle, les dérivés diacylglycérol, c'est-à-dire ceux pour lesquels le substituant succinyle porte en bout de chaîne, un glycérol portant lui-même des chaînes grasses est particulièrement utile du fait de sa grande biodisponibilité.

En cffet, la présence des chaînes grasses permet une bonne résorption lymphatique. De même, les dérivés du type oléyle sont fortement résorbés, ce qui leur donne une grande biodisponibilité.

D'autres caractéristiques et avantages de l'invention apparaîtront à la lecture des exemples ci-après.

Dans tout ce qui suit, le biradical [4-[2-(.2,4-dichlorophényl)-2-(1H-imidazol-1-yl-méthyl)-1,3 dioxolan-4-ylméthoxy]phényl] sera représenté par -[A]-, le composé Cis-1-[A]- pipérazine étant préparé par désacétylation du kétoconazole.

EXEMPLE I :

Cis-4-[A]-1-(glycyl)pipérazine (composé n° 1)

A 1 g (20,4 mmoles) de Cis-1-[A]-pipérazine en suspension dans 100 ml d'acétate d'éthyle, on ajoute 0,88 g (22,4 mmole) de N-fluorenylméthyloxycarbonylglycinate de N-hydroxy succimide. La solution agitée durant une nuit à température ambiante devient progressivement limpide. Après évaporation du solvant, le produit est lavé à l'eau et extrait au chloroforme. La phase organique est lavée à la saumure, séchée sur sulfate de sodium, filtrée et évaporée. Le produit est purifié sur gel de silice en utilisant comme éluant un mélange chloroforme/méthanol : 96/4. Les fractions pures sont rassemblées et l'éluant évaporé conduisant à un solide blanc recristallisé dans un mélange dichlorométhane/hexane. On isole ainsi 1,04 g (69 %) de cis-4-[A]-1-(N-fluorenylméthyloxycarbonyl-glycyl)-piperazine.

IR (KBr) : $\nu(cm^{-1})$ : 3310, 1720, 1650, 1625, 1510

0,72 g (9,37 mmoles) du composé précédent sont dissous dans 10 ml de pipéridine fraîchement distillée sur KOH. Le mélange est agité durant 1 h à température ambiante. La pipéridine est évaporée sans vide, le solide blanc obtenu lavé plusieurs fois à l'éther et purifié sur une colonne d'alumine en utilisant comme éluant un mélange chloroformé/méthanol : 9/1. Le produit est ensuite recristallisé dans un mélange dichlorométhane/éther. On isole ainsi 0,41 g(80%) de composé du titre (n°1).

 

Fus. : 152°C
IR(KBr : ν($cm^{-1}$) : 3360, 3320, 1655, 1510
Masse : m/e : 545 ($M^+$)

EXEMPLE II :

Cis-4-[A]-1-(alanyl)pipérazine (composé n°2)

En suivant le même mode opératoire que celui décrit à l'exemple I, on isole au départ de cis-1-[A]-pipérazine et de N-fluorenylméthyloxycarbonyl alaninate de N-hydroxy succimide et après déprotection de la fonction amine à l'aide de pipéridine 61 % du composé du titre(n°2)

IR (KBr) : ν($cm^{-1}$) : 3360, 1640, 1510
Masse m/e : 559 ($M^+$)

EXEMPLE III :

Cis-4-[A]-1-(N-glycylglycyl)pipérazine (composé n° 3)

En suivant le même mode opératoire que celui décrit en I,on isole au départ du composé n° 1 et de N-fluorenylméthyloxycarbonyl glycinate de N-hydroxy-succinimide après déprotection de la fonction amine à l'aide de pipéridine 55 % de composé du titre (n° 3).

IR(KBr) : ν($cm^{-1}$) : 3320, 1665, 1645, 1510.
Masse m/e : 602 ($M^+$)

EXEMPLE IV :

Cis-4-[A]-1(succinyl) pipérazine (composé n° 4)

A 0,489 g (1 mmole) de Cis-1-[A]-pipérazine en suspension dans 10 ml de diméthylformamide, on ajoute à température ambiante 0,11 g (1,1mmole) d'anhydride succinique en solution dans 2 ml de diméthylformamide. La solution est agitée durant 2 h à température ambiante et devient progressivement limpide. Le solvant est évaporé sous vide et le solide obtenu recristallisé dans l'isopropanol. On isole ainsi 0,494 g (86,5 %) du composé du titre (n° 4).

IR(KBr)ν : 1710, 1650, 1510 $cm^{-1}$
Masse : m/e décomposition

EXEMPLE V :

Cis-4-[ A ]-1-(N-succinylglycyl)pipérazine (composé n° 5)

En suivant le même mode opératoire que celui décrit à l'exemple IV, on isole au départ du composé n° 1 et d'anhydride succinique, en utilisant la diméthylformamide comme solvant, 79 % du composé du titre (n° 5).

IR(KBr)ν($cm^{-1}$) : 3345, 1720, 1655, 1630, 1510 $cm^{-1}$
Masse M/e : décomposition

EXEMPLE VI :

Cis-4-[ A ]-1-(N-acétylglycyl) pipérazine (composé n° 6)

En suivant le même mode opératoire que celui décrit à l'exemple IV, on isole au départ du composé n° 1 et d'anhydride acétique, le solvant étant la diméthylformamide, 80,7 % du composé du titre (n° 6).

Ir(KBr) : ν $cm^{-1}$ : 3310, 1645, 1510
Masse : m/e : 587 ($M^+$)

EXEMPLE VII :

Cis-4-[ A ]-1-(oléoyl)pipérazine (composé n° 7)

2,5 g (5,11 mmoles) de cis-1-[ A ]-piperazine sont dissous dans 100 ml de chloroforme sec. On ajoute à cette solution 0,51 g (5,11 mmoles) de triéthylamine Le mélange est refroidi dans la glace et 1,67 ml (5,11 mmole) de chlorure d'oléoyle sont ajoutés goutte à goutte. Le mélange réactionnel est ensuite agité durant 2 h à température ambiante. Après

évaporation du solvant, le produit est purifié par chromatographie sur gel de silice en utilisant comme éluant un mélange chloroforme/méthanol : 9/1. Les fractions pures sont rassemblées et l'éluant évaporé. On isole ainsi 3,2 g (84 %) du composé du titre (n° 7) sous la forme d'une huile.

IR (Film) : ν(cm$^{-1}$) : 1645, 1510
Masse : m/e : 753 ($M^+$)

EXEMPLE VIII :

Cis-4-[A]-1-(N-oléoylglycyl)pipérazine (composé n° 8)

A 0,138 g (0,25 mmole) du composé n° 1 en solution dans 3 ml de chloroforme sec, on ajoute 0,035 ml (0,25 mmole) de triéthylamine. Après avoir refroidi le mélange dans un bain de glace, on y ajoute 0,082 ml (0,25 mmole) de chlorure d'oléoyle. La solution est ensuite agitée durant 1 h à température ambiante. Après évaporation du solvant, le produit est purifié et séparé de l'hydrochlorure de triéthylamine par chromatographie sur gel de silice en utilisant comme éluant un mélange chloroforme/méthanol : 9/1. Les fractions pures sont rassemblées, l'éluant évaporé et l'huile obtenue triturée dans l'hexane donne une poudre blanche. On isole ainsi 0,177 g (86,7 %) du composé du titre (n° 8) cristallisé dans un mélange dichlorométhane/hexane.

IR(KBr) : ν(cm)$^{-1}$ : 3340, 1660, 1640, 15106
Masse : m/e 810 ($M^+$)

EXEMPLE IX :

Test d'activité des composés

L'activité des nouveaux dérivés selon l'invention a été testée *in vitro* en déterminant la concentration minimale

inhibitrice (CMI) des molécules et leur activité fongicide (concentration minimale fongicide CMF) ou bactéricide (composition minimale bactéricide CMB). Les CMF et CMB sont définies comme la dose minimale qui tue 99,9 % des organismes contenus dans l'inoculum. Deux espèces de levures pathogènes pour l'homme ont été utilisées : Candida albicans ATCCe10231 et Candida tropicalis ATCC 13803. Deux espèces bactériennes sont inclues dans le criblage : Staphylococcus aureus ATCC 25.923 et Salmomella typhimurium LT2 souche Demerec. La CMI est mesurée apres une nuit de culture en milieu liquide par la méthode de microtitration dans des boîtes à 96 puits dans une gamme de concentrations allant de 100 µM à 0,0008 µM. L'inoculum contient de $10^5$ à $10^6$ germes/ml. Les milieux de culture sont d'une part le RPMI additionné de 20 % de serum de veau foetal (inactivé une heure à 56°) pour mesurer l'activité fongistatique et d'autre part le Mueller-Hinton pour déterminer le pouvoir bactériostatique des molécules néosynthétisées. La CMF et la CMB pour chacune de ces molécules, sont obtenues en subcultivant sur milieu solide YM agar (levures) ou Mueller-Hinton agar (bactéries) pendant une nuit à 37°, un échantillon contenant $10^3$ organismes à partir de chaque puits négatif pour la croissance des germes.

Les résultats figurent aux tableaux I et II ci-après.

Les produits de référence sont le miconazole et la Fungizone® (Amphotéricine B).

TABLEAU I

| COMPOSE | CMI (µM) | | | |
|---|---|---|---|---|
| | C.albicans | C.tropicalis | S.aureus | S.typhimurium |
| Cis-1-[A]-pipérazine | 0,13 | 0,117 | 3,65 | >100 |
| Kétoconazole | 0,053 | 0,049 | 20,44 | >100 |
| n° 1 | 0,16 | 0,119 | 11,98 | |
| n° 2 | 0,006 | 0,006 | | |
| n° 3 | 0,48 | 0,48 | | |
| n° 4 | 19,82 | 15,23 | 50,78 | |
| n° 5 | 25 | 25 | 50 | |
| n° 6 | 3,13 | 0,78 | | |
| n° 7 | 10,2 | 8,78 | >100 | >100 |
| n° 8 | 31,25 | 53,13 | | |
| PRODUITS DE REFERENCE | | | | |
| Miconazole | 0,048 | 0,78 | 1,56 | |
| Fungizone® | 0,33 | 0,325 | >100 | >100 |

TABLEAU II

| COMPOSE | CMF (µM) | | | |
|---|---|---|---|---|
| | C.albicans | C.tropicalis | S.aureus | S.typhimurium |
| Cis-1-[A]-pipérazine | 2,35 | 1,58 | 25 | |
| kétoconazole | 0,22 | 4,88 | 50 - >100 | |
| n° 1 | 2,38 | 1,3 | 25 | |
| n° 4 | 25 | 50 | >100 | |
| n° 6 | 6,25 | 12,5 | | |
| n° 7 | 125 | 250 | >100 | |
| n° 8 | 62,5 | >100 | | |
| Fungizone® | 0,83 | 3,17 | 25 - >25 | >100 |

EXEMPLE X :

Résorption

Des rats sont administrés par voie gastrique avec 100 µmoles/0,5 ml de DMSO par 100 g de poids corporel, soit de kétoconazole (produit de référence) soit d'oléoylkétoconazole (composé n° 7). Les animaux sont sacrifiés au $CO_2$, 1 h après l'administration. Le sang est prélevé par ponction cardiaque et centrifugé pendant 15 minutes à 2000 RPM pour obtenir le plasma.

Les plasmas sont dilués de 2 en 2 fois dans une solution de NaCl 0,9 %. De chaque dilution, 10 µl sont prélevés et déposés sur des disques de papier Whatman. Les disques sont placés sur des boîtes de Pétri contenant 15 ml de casitone agar surmonté d'une couche de 3 ml d'un agar moins dense mélangé à 0,1 ml d'une culture de nuit de Candida tropicalis. Après une nuit d'incubation, les zones d'inhibition de croissance autour des disques sont mesurées et comparées à une courbe étalon de kétoconazole établie dans les mêmes conditions.

Une concentration plasmatique correspondant à 0,44 µM en antifongique est obtenue si les rats ont reçu le kétoconazole et de 57,3 µM si les rats ont été injectés avec de l'oléoylkétoconazole.

EXEMPLE XI :

Effet des dérivés du kétoconazole sur la synthèse des stéroïdes in vitro et effet anti-tumoral

L'effet anti-tumoral des dérivés du kétoconazole sur le cancer prostatique androgénodépendant est mis en évidence ci-après. Le kétoconazole et ses dérivés diminuent le taux plasmique de la testostérone par inhibition de certains enzymes impliqués dans sa biogénèse.

La progestérone constitue un intermédiaire-clef dans la biogénèse des hormones stéroïdes puisqu'elle conduit notamment à l'estradiol dans l'ovaire et à la testostérone dans les testicules.

Le pouvoir inhibiteur du kétoconazole et de ses dérivés sur la chaîne enzymatique de la biogénèse des hormones stéroïdes peut être évalué par la mesure de la transformation du cholestérol en progestérone. Nous décrivons ci-dessous un dosage permettant de quantifier cette propriété.

Des cellules de Leydig tumorales de souris (I-10, ATCC n° CCL83) ont été cultivées dans un milieu HAM's F10 additionné de 15% de sérum de cheval et de 2,5% de sérum de veau foetal. Ces cellules ont la propriété de sécréter de la progestérone ainsi qu'un produit de réduction de cette dernière qui n'est plus métabolisé, la 20 alpha-hydroxy-4-pregnen-3 one. Cette sécrétion de stéroïdes est induite par addition d'AMPc sous sa forme d'ester dibutyrylique (N6,2'O-dibutyryl adénosine 3',5'-cyclic monophosphate sodium salt monohydrate (Sigma)). Les cellules sont subcultivées dans des boîtes falcon de 25 $cm^2$ et maintenues sous $CO_2$ (10%). Lorsque celles-ci sont confluentes ($\pm$ 1 mg/ml en protéines), le milieu est remplacé par du milieu frais contenant du dibutyryle AMPc ($1x10^{-4}$ M). Après 1 heure d'incubation avec l'AMPc, le kétoconazole ou ses dérivés sont ajoutés ($9,4x10^{-7}$ M) au milieu de culture. Les surnageants sont prélevés après 3 heures d'incubation supplémentaires et serviront à l'analyse des stéroïdes par HPLC. Les cellules sont rincées au PBS (phosphate buffered saline : NaCl 0,15 M, KCl 2,7 mM, $Na_2HPO_4$-$KH_2PO_4$ 3 mM pH 7,4) et après traitement au DOC (acide désoxycholique), on procède au dosage des protéines par la méthode de Lowry.

Pour le dosage des stéroïdes, 2 ml de milieu de culture sont mis en présence de 5 ml d'éther. Les tubes sont agités au vortex pendant 2 minutes. Après congélation des phases aqueuses dans un bain isopropanol/carboglace, la phase éthérée est prélevée et l'éther est évaporé sous courant d'azote à 30°C. L'extrait sec est ensuite dilué dans 150 µl d'eau et 150 µl d'acétonitrile et centrifugé à 13.000 rpm pendant 10 minutes. 30 µl du surnageant sont injectés en HPLC sur une µBondapack C18 de 4 mm de diamètre et de 30 cm de long (WATERS). La phase mobile est composée de 38% d'eau, de 31% en acétonitrile et de 31% en méthanol. Le débit est de 2 ml/min, et la lecture s'effectue à 245 nm.

TABLEAU 3

| Composé | Progestérone | | 20 alpha-OH-4-pregnen-3 one | |
|---|---|---|---|---|
| | (1) | (2) | (1) | (2) |
| Kétoconazole | 1878 | 53 | 1296 | 74 |
| Cis-1-/A/-pipérazine | 2783 | 78 | 1461 | 84 |
| N° 2 | 1939 | 55 | 1299 | 74 |
| N° 7 | 2754 | 78 | 1497 | 86 |
| N° 9 | 2231 | 63 | 1725 | 99 |
| N° 10 | 323 | 9 | 605 | 35 |
| Contrôle (+ AMP) | 3546 | 100 | 1744 | 100 |
| Basale (- AMP)* | 544 | - | 785 | - |

(1) ng de stéroïdes/mg de protéines cellulaires/4 heures

(2) % d'activité enzymatique par rapport au contrôle, c'est-à-dire des cellules induites par l'AMPc mais en absence de l'inhibiteur

* la situation basale est celle obtenue lorsque les cellules n'ont pas été induites par l'AMPc

Cis-4-/A/-1(leucyl) pipérazine (composé n° 9)

En suivant le même mode opératoire que celui décrit à l'exemple 1, on isole au départ de Cis-1-/A/-pipérazine et de N-fluorenylméthyloxycarbonyl leucinate de N-hydroxysuccinimide et après déprotection de la fonction amine à l'aide de pipéridine 55% du composé du titre (n° 9).

IR (KBr) : ν ($cm^{-1}$) : 3360, 1640, 1510

Cis-4-/A/-1-(phénylalanyl) pipérazine (composé n° 10)

En suivant le même mode opératoire que celui décrit à l'exemple 1, on isole au départ de Cis-1/A/-pipérazine et de N-fluorenylméthoxycarbonyl phénylalaninate de N-hydroxysuccinimide et après déprotection de la fonction amine à l'aide de pipéridine 33% du composé du titre (n° 10).

IR (KBr) : ν ($cm^{-1}$) : 3360, 1640, 1510

Masse : (DCI/Acétone) : 636 ($M^+$ + 1)

## REVENDICATIONS

1. Composés de formule

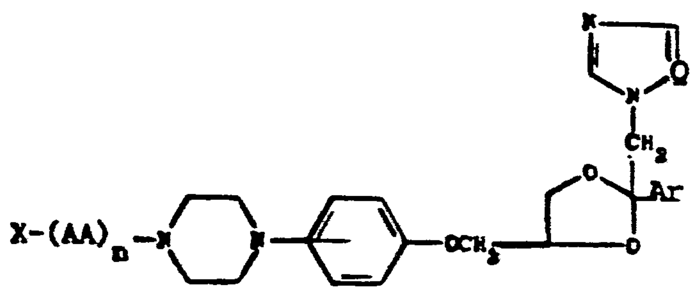

(I)

dans laquelle

Q est N ou CH,

Ar est un radical choisi parmi phényle et thienyle éventuellement substitué, les substituants pouvant être de un à trois substituants halo, alkyle inférieur ou alkoxy inférieur,

-AA- représente un radical divalent correspondant à un amino acide entrant dans la constitution des protéines, certaines fonctions de ce radical pouvant être protégées, ou bien représente un radical diacyle,

n est un entier de 0 à 4 inclus, et

X représente H, OH ou Y, $-\underset{\underset{O}{\|}}{C}-Y$, -O-Y, ou 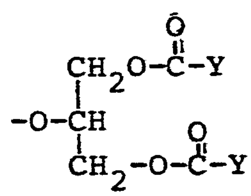 ,où

lorsque n est égal à 1,2,3 ou 4, alors Y est un radical hydrocarboné ayant de 1 à 20 atomes de carbone,

lorsque n est égal à 0, alors Y est un radical hydrocarboné ayant de 7 à 20 atomes de carbone,

ainsi que les sels et les isomères de ces composés.

2. Composés selon la revendication 1 de formule :

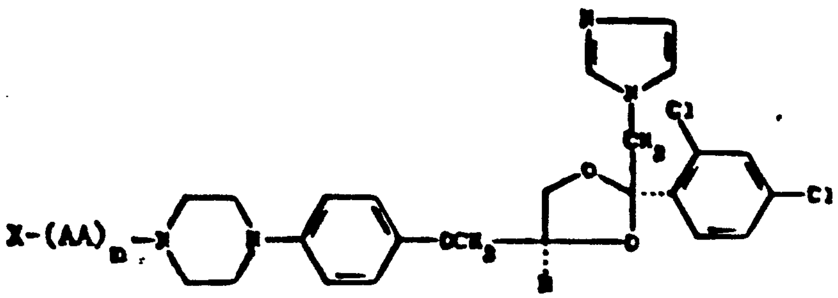 (Ia)

dans laquelle
-(AA)- représente 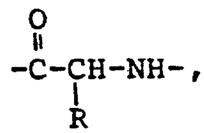,
où R représente les substituants latéraux en α du groupe amino des acides aminés, ou le radical diacyle succ
n représente un entier de O à 4 inclus;
X représente H, Y, 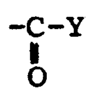, OY ou

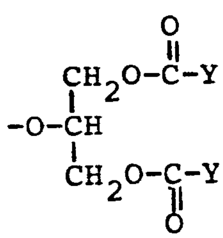, où

Y représente un radical hydrocarboné en $C_7$ à $C_{20}$, alkyle ou alcényle,
et succ représente le radical 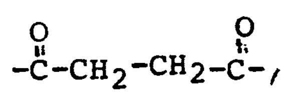,

et lorsque n ≠ O alors Y peut être alkyle inférieur, leurs sels pharmaceutiquement acceptables et leurs formes isomères.

3. Composés selon la revendication 2, où R représente H ou $CH_3$ et leurs sels pharmaceutiquement acceptables.

4. Composés selon la revendication 1,2 ou 3, où $-(AA)_n-X$ a les significations :

$-COCH_2NH_2$

$-COCHNH_2$ avec $CH_3$ sur le carbone (–COCH($CH_3$)NH_2)

$-COCH_2NHCOCH_2NH_2$

$-COCH_2CH_2COOH$

$-COCH_2NHCOCH_2CH_2COOH$

$-COCH_2NHCOCH_3$

$-CO(CH_2)_7CH{=}CH(CH_2)_7CH_3$

$-COCH_2NHCO(CH_2)_7CH{=}CH(CH_2)_7CH_3$.

et leurs sels pharmaceutiquement acceptables.

5. Procédé de préparation de composés selon la revendication 1, caractérisé en ce que on fait réagir le composé de formule

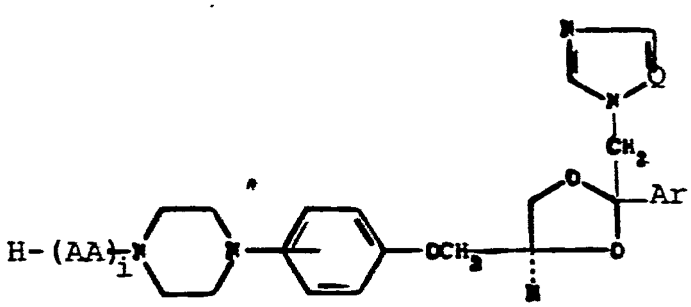

II

avec un acide de la formule

$$X-(AA)_{n-i}-H$$ III

où i est un entier et $0 \leqslant i \leqslant n$,

0236187

6. Procédé de préparation de composés selon la revendication 1, caractérisé en ce que
a) on fait réagir le composé de formule

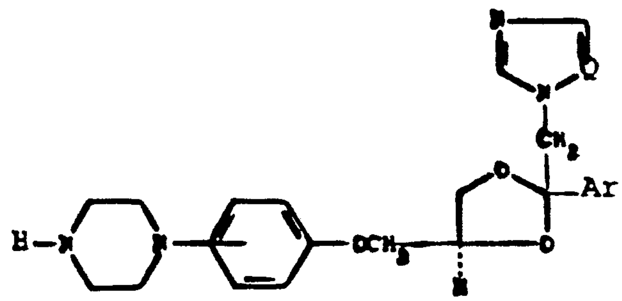

IV

avec un acide de la formule

X-(AA)$_n$-H V

ou un dérivé réactif de cet acide carboxylique, ou

b) on fait réagir le composé de formule

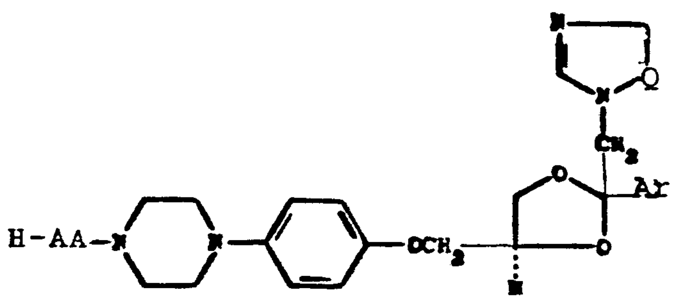

VI

avec un composé de formule

X-(AA)$_{n-1}$-H VII

ou un dérivé réactif de ce composé, éventuellement en présence d'un auxiliaire de réaction, dans un solvant approprié et dans des conditions de température appropriées, les composés VI et VII étant éventuellement sous forme de dérivé réactif de la fonction acide ou aminé et les fonctions non réactives pouvant être protégées.

7. A titre de médicament nouveau, le composé selon l'une des revendications 1 à 4.

8. Compositions bactéricides et/ou fongicides et/ou anticancéreuses comportant au moins une quantité efficace d'un composé selon l'une des revendications 1 à 4 ou un sel d'addition acide ou un de leur stéréoisomère, et un support acceptable.

9. Composition pharmaceutique comportant au moins une quantité efficace d'un composé selon l'une des revendications 1 à 4, un sel pharmaceutiquement acceptable ou un de leur stéréoisomère, et un support pharmaceutiquement acceptable.

10. Application des composés selon l'une des revendications 1 à 4, leurs sels d'addition acide ou leurs stéréoisomères comme agent fongicide et/ou bactéricide et/ou anticancéreux.

0236187

Office européen des brevets

# RAPPORT DE RECHERCHE EUROPEENNE

Numéro de la demande

EP 87 40 0225

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| D,Y | EP-A-0 006 722 (JANSSEN PHARMACEUTICA N.V.) * Revendications * | 1,5-10 | C 07 K 5/06<br>C 07 D 405/14<br>A 61 K 31/495 |
| | --- | | |
| Y | EP-A-0 050 298 (HOECHST AG) * Revendications * | 1,5-10 | |
| | ----- | | |

DOMAINES TECHNIQUES RECHERCHES (Int. Cl.4)

C 07 K 5/00
C 07 D 405/00
C 07 D 233/00
C 07 D 249/00
A 61 K 31/00

Le présent rapport de recherche a été établi pour toutes les revendications

| Lieu de la recherche | Date d'achèvement de la recherche | Examinateur |
|---|---|---|
| LA HAYE | 08-05-1987 | CHOULY J. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82